# FASCICULE DE BREVET EUROPEEN

(11) **EP 0 637 579 B1**
(45) Date de publication et mention de la délivrance du brevet: **29.07.1998**
(21) Numéro de dépôt: 94202126.2
(22) Date de dépôt: 21.07.1994
(51) Int. Cl.: C07C 17/00, C07C 17/20, C07C 19/08

(54) **Procédé pour la préparation de 1-chloro-1-fluoroéthane et/ou de 1,1-difluoroéthane**
Verfahren zur Herstellung von 1-Chlor-1-Fluorethan und/oder 1,1-Difluorethan
Process for the preparation of 1-chloro-1 fluoroethane and/or 1,1-difluoroethane

(30) Priorité: 04.08.1993 BE 9300816
(43) Date de publication de la demande: 08.02.1995
(73) Titulaire: SOLVAY (Société Anonyme), B-1050 Bruxelles (BE)
(72) Inventeur: Pennetreau, Pascal, B-1330 Rixensart (BE); Janssens, Francine, B-1800 Vilvoorde (BE)
(74) Mandataire: Anthoine, Paul

(56) Documents cités:
- FR-A- 2 019 507
- FR-A- 2 365 542
- GB-A- 921 254

## Description

La présente invention concerne un procédé pour la préparation de 1-chloro-1-fluoroéthane (HFA-151a) et/ou de 1,1-difluoroéthane (HFA-152a), au départ de chlorure de vinyle, réalisé en phase liquide dans un solvant organique.

Il est connu de préparer du 1-chloro-1-fluoroéthane par réaction entre du chlorure de vinyle et du fluorure d'hydrogène en phase liquide.

Par exemple, dans le brevet DE 859887, le 1-chloro-1-fluoroéthane est obtenu par réaction entre le chlorure de vinyle et le fluorure d'hydrogène, en l'absence de catalyseur, simplement par introduction des réactifs dans un autoclave, dans lequel ils sont chauffés modérément sous pression autogène. Ce procédé conduit toutefois à la formation d'une quantité importante de sous-produits huileux.

Le brevet US 2495407 enseigne la préparation de 1-chloro-1-fluoroéthane au départ de chlorure de vinyle en présence d'halogénure d'étain. Dans les différents exemples, les réactifs sont chargés dans un autoclave avec la quantité désirée de catalyseur, puis soumis à une température proche de 40 °C, sous pression autogène. Ce brevet rapporte également des essais infructueux réalisés en présence d'acide acétique ou d'acétone comme solvant.

On a cependant observé que ces procédés connus conduisent à la formation, en quantité parfois très importante, de sous-produits halogénés lourds, formés principalement d'oligomères du chlorure de vinyle, en partie fluorés. La formation de ces sous-produits affecte gravement les rendements de la réaction en 1-chloro-1-fluoroéthane et en 1,1-difluoroéthane. En outre, une destruction propre de ces sous-produits halogénés nécessite la mise en oeuvre de techniques délicates et très coûteuses.

On a proposé, dans le brevet FR 1396251, un procédé de fabrication de dérivés halogénés de l'éthane, qui évite la formation parasite de dérivés halogénés lourds en quantités importantes. A cet effet, on introduit de façon continue, à la pression atmosphérique, un halogénure de vinyle à l'état gazeux dans un réacteur contenant un milieu liquide formé essentiellement de fluorure d'hydrogène anhydre, maintenu à une température comprise entre -15 et +19 °C. Dans le but d'accélérer la réaction ou de réduire la tension de vapeur du fluorure d'hydrogène, il est envisagé dans ce document d'ajouter au fluorure d'hydrogène diverses substances acides ou inertes miscibles à celui-ci, en une quantité pondérale inférieure ou égale à la quantité de fluorure d'hydrogène. Dans le cas où on utilise du chlorure de vinyle, ce procédé connu donne lieu à la production conjointe de 1-chloro-1-fluoroéthane et de 1,1-difluoroéthane. Ce procédé apparaît toutefois peu intéressant en pratique. Il nécessite en effet de travailler avec une quantité importante de fluorure d'hydrogène dans le réacteur, ce qui implique des problèmes aigus de sécurité. Ce procédé n'apparaît en outre pas adapté à une production modulable de 1-chloro-1-fluoroéthane et de 1,1-difluoroéthane, qui soit industriellement exploitable.

La présente invention a pour but de remédier aux inconvénients précités des procédés connus et de procurer un procédé de préparation de 1-chloro-1-fluoroéthane et/ou de 1,1-difluoroéthane au départ de chlorure de vinyle, qui possède une productivité élevée et qui permet de moduler dans une large gamme les productions relatives du 1-chloro-1-fluoroéthane et du 1,1-difluoroéthane, tout en limitant la formation parasite de sous-produits halogénés lourds.

A cet effet, l'invention concerne un procédé de fabrication de 1-chloro-1-fluoroéthane et/ou de 1,1-difluoroéthane par réaction entre du fluorure d'hydrogène et du chlorure de vinyle en phase liquide, qui se caractérise en ce qu'on introduit le fluorure d'hydrogène et le chlorure de vinyle dans un solvant organique constitué d'au moins un hydrocarbure halogéné saturé.

L'hydrocarbure halogéné saturé est de préférence sélectionné parmi les hydrocarbures chlorés, fluorés ou chlorofluorés contenant de 1 à 8 atomes de carbone.

L'hydrocarbure halogéné saturé du solvant peut être un hydrocarbure halogéné extérieur à la réaction, c'est-à-dire un composé différent de ceux qui sont formés au départ du chlorure de vinyle. Des hydrocarbures halogénés extérieurs à la réaction qui conviennent dans le procédé selon l'invention sont notamment le trichlorométhane, le tétrachlorométhane, le 1,2-dichloroéthane, le 1,1,2-trichloroéthane, le 1,1,2,2-tétrachloroéthane et le 1,2,3-trichloropropane. Parmi ces composés, le 1,2-dichloroéthane et le 1,2,3-trichloropropane sont préférés. Le recours à de tels hydrocarbures halogénés extérieurs à la réaction peut s'avérer intéressant lorsque le procédé est réalisé de manière discontinue.

De manière avantageuse, on utilise, pour l'hydrocarbure halogéné saturé du solvant, un hydrocarbure halogéné saturé du procédé. De tels hydrocarbures halogénés sont notamment le 1-chloro-1-fluoroéthane, le 1,1-difluoroéthane, le 1,1-dichloroéthane et des composés contenant de 4 à 8 atomes de carbone, tels que le 1,3-dichloro-1-fluorobutane. De manière tout particulièrement préférée, le solvant est constitué d'un mélange d'hydrocarbures halogénés produits dans le procédé.

Dans la suite de l'exposé, l'ensemble du milieu liquide formé du solvant organique, du fluorure d'hydrogène et du chlorure de vinyle mis en oeuvre, des produits formés par la réaction du fluorure d'hydrogène et du chlorure de vinyle et, le cas échéant, des additifs habituellement utilisés dans les procédés de fluoration des hydrocarbures halogénés, tels que des catalyseurs, est dénommé mélange réactionnel.

Selon l'invention, pour observer un effet bénéfique sur la quantité de sous-produits lourds formés, le mélange réactionnel doit contenir, à tout moment, au moins 55 % en poids de solvant. De préférence, le mélange réactionnel contient au moins 70 % en poids de solvant. De manière particulièrement préférée, il en contient au moins 80 % en poids. Les meilleurs résultats sont obtenus avec une teneur en solvant dans le mélange réactionnel supérieure à 90 % en poids. En général, la teneur en solvant dans le mélange réactionnel est inférieure à 99,5 %. De préférence, elle est inférieure à 98,5 %.

Conformément au procédé selon l'invention, on introduit le chlorure de vinyle et le fluorure d'hydrogène dans le solvant organique. De manière avantageuse, on règle l'introduction du chlorure de vinyle pour que, à tout moment, la teneur en chlorure de vinyle soit inférieure à 15 % du poids du mélange réactionnel. En effet, lorsque la teneur en chlorure de vinyle est plus élevée, on observe la formation de quantités importantes de sous-produits lourds. De manière particulièrement avantageuse, on travaille avec une teneur en chlorure de vinyle inférieure à 10 % en poids. De manière tout particulièrement avantageuse, elle ne dépasse à aucun moment 5 % en poids. Plus la teneur en chlorure de vinyle dans le milieu réactionnel est faible, moins on forme de sous-produits lourds. En pratique, pour atteindre une productivité suffisante, on travaille généralement avec une teneur en chlorure de vinyle dans le mélange réactionnel au moins égale à 0,1 % en poids, de préférence au moins égale à 0,5 % en poids. De manière avantageuse, on règle l'introduction du fluorure d'hydrogène pour que, à tout moment, la teneur en fluorure d'hydrogène soit inférieure à 30 % du poids du mélange réactionnel. De manière particulièrement avantageuse, on travaille avec une teneur en fluorure d'hydrogène inférieure à 15 % en poids. En règle générale, la teneur en fluorure d'hydrogène dans le mélange réactionnel est au moins égale à 0,3 % en poids. De préférence, elle est au moins égale à 1 % en poids.

Dans le procédé selon l'invention, on introduit avantageusement le fluorure d'hydrogène dans un rapport molaire fluorure d'hydrogène / chlorure de vinyle au moins égal à 1. Le plus souvent, ce rapport molaire ne dépasse pas 20. Par un réglage approprié du rapport molaire entre le fluorure d'hydrogène et le chlorure de vinyle introduits, on parvient à réguler dans une large gamme les proportions relatives de 1-chloro-1-fluoroéthane et de 1,1-difluoroéthane produits. Lorsque le produit désiré est le 1-chloro-1-fluoroéthane, on travaille de préférence avec un rapport molaire de 1,1 à 4. Lorsque le produit désiré est le 1,1-difluoroéthane, on travaille de préférence avec un rapport molaire de 2 à 15.

Le mélange réactionnel peut contenir divers additifs, par exemple un co-solvant polaire inerte tel que la N-méthylpyrrolidone ou un catalyseur d'hydrofluoration. La présence d'un catalyseur est facultative lorsque le produit désiré est le 1-chloro-1-fluoroéthane. Elle est souhaitable pour obtenir du 1,1-difluoroéthane dans des conditions industriellement exploitables. Comme catalyseurs utilisables, on peut citer les dérivés des métaux choisis parmi les métaux des groupes IIIa, IVa, IVb, Va, Vb et VIb du tableau périodique des éléments et leurs mélanges. On retient plus spécialement les dérivés de titane, de vanadium, de tantale, de molybdène, de tungstène, d'étain et d'antimoine. Les dérivés de l'étain, du molybdène et du tungstène conduisent à la formation de 1,1-difluoroéthane en quantités importantes et sont dès lors préférés lorsqu'on désire préparer ce produit. Dans ce cas, les dérivés de l'étain conviennent particulièrement bien. Par contre, les dérivés du vanadium, du chrome et du titane sont préférés lorsque le 1-chloro-1-fluoroéthane est le produit recherché. Comme dérivés des métaux, on utilise de préférence les halogénures, tels que les chlorures, les fluorures et les chlorofluorures, ainsi que les oxydes et les oxyhalogénures. Des catalyseurs particulièrement préférés pour préparer le 1,1-difluoroéthane par le procédé selon la présente invention sont les chlorures, les fluorures et les chlorofluorures d'étain, le SnCl₄ étant tout particulièrement préféré. Des catalyseurs particulièrement préférés pour préparer le 1-chloro-1-fluoroéthane par le procédé selon la présente invention sont les chlorures, les fluorures et les chlorofluorures de vanadium ou de chrome, le VF₄ étant tout particulièrement préféré.

Lorsqu'un catalyseur est utilisé dans le procédé selon l'invention, la quantité de catalyseur mise en oeuvre peut varier dans de larges limites. Habituellement, le catalyseur est utilisé à raison de 0,001 à 2 mole de catalyseur par kg de mélange réactionnel et, de préférence, de 0,01 à 1 mole par kg.

Généralement, la température à laquelle la réaction est réalisée est au moins de 40 °C et ne dépasse pas 130 °C. De préférence, elle est d'au moins 50 °C et ne dépasse pas 120 °C.

La pression est choisie de manière à maintenir le mélange réactionnel sous forme liquide. Elle varie en fonction de la température du mélange réactionnel. Elle est généralement au moins égale à 2 bar. Le plus souvent, elle ne dépasse pas 50 bar.

Le procédé selon l'invention peut être réalisé selon un mode discontinu ou continu. Un mode de réalisation avantageux du procédé, qui permet de maintenir dans le mélange réactionnel une teneur adéquate en solvant organique consiste à introduire le chlorure de vinyle et le fluorure d'hydrogène réactifs en continu dans le réacteur contenant le solvant organique et à soutirer le produit recherché, c'est-à-dire le 1-chloro-1-fluoroéthane et/ou le 1,1-difluoroéthane, en continu hors du mélange réactionnel. Dans ce mode de réalisation avantageux, le solvant peut être constitué, en tout ou en partie par des produits formés dans le procédé. En particulier, du 1-chloro-1-fluoroéthane et/ou du 1,1-difluoroéthane produits peuvent être maintenus dans le réacteur en tant que solvant. Du 1,1-dichloroéthane risque parfois d'être formé de manière transitoire par réaction entre du chlorure d'hydrogène produit dans le procédé et le chlorure de vinyle. Maintenu dans le réacteur comme solvant, il est progressivement transformé en 1-chloro-1-fluoroéthane et/ou en 1,1-difluoroéthane. Le cas échéant, des sous-produits lourds, c'est-à-dire des composés contenant généralement de 4 à 8 atomes de carbone et présentant un point d'ébullition supérieur à celui du produit recherché, tel que le 1,3-dichloro-1-fluorobutane, peuvent également être formés en faibles quantités dans le procédé. Dans ce mode de réalisation avantageux du procédé, le solvant organique peut donc être constitué d'un mélange comprenant du 1-chloro-1-fluoroéthane, du 1,1-difluoroéthane, du 1,1-dichloroéthane et des sous-produits lourds. Afin d'éviter que les sous-produits lourds ne s'accumulent en quantités trop importantes dans le réacteur, par exemple en quantités dépassant 60 % du poids du mélange réactionnel, on peut, lorsque le réacteur est en régime, en soutirer une faible partie, par exemple en procédant régulièrement à une purge.

Le soutirage du produit recherché hors du mélange réactionnel peut se faire de différentes manières. Ainsi, on peut prélever en continu, sous forme liquide, une partie du mélange réactionnel et la soumettre à une séparation, par exemple par distillation, de manière à recueillir séparément, d'une part, le produit recherché et, d'autre part, le reste du mélange réactionnel, que l'on peut recycler au réacteur. Dans ce mode de réalisation bien adapté à la synthèse du 1-chloro-1-fluoroéthane, on utilise de préférence des pressions élevées, comprises par exemple entre 10 et 50 bar.

Selon un mode de réalisation particulièrement préféré de l'invention appliqué à la synthèse du 1,1-difluoroéthane, on réalise dans le réacteur, une température et une pression telles que le 1,1-difluoroéthane formé quitte le mélange réactionnel en continu, sous forme gazeuse. De manière avantageuse, on soutire par la même voie tout ou partie du chlorure d'hydrogène formé par la réaction. Dans ce mode de réalisation particulièrement préféré de l'invention, le mélange gazeux que l'on recueille contient, outre le 1,1-difluoroéthane et le chlorure d'hydrogène, un peu de fluorure d'hydrogène et de chlorure de vinyle non convertis, un peu de 1-chloro-1-fluoroéthane et de 1,1-dichloroéthane éventuellement co-produits et, lorsque l'hydrocarbure halogéné est un composé extérieur à la réaction, éventuellement un peu de cet hydrocarbure halogéné. Ce mélange gazeux peut être soumis à une ou plusieurs techniques de séparation connues en soi, par exemple par distillation, de manière à recueillir le 1,1-difluoroéthane sous forme pure, tandis que les autres produits, le chlorure d'hydrogène excepté, peuvent être recyclés au réacteur.

Selon ce mode de réalisation particulièrement préféré de l'invention, la température et la pression de réaction sont réglées de manière, d'une part, à assurer le maintien en phase liquide du mélange réactionnel et, d'autre part, à permettre au 1,1-difluoroéthane de quitter le mélange réactionnel sous forme gazeuse, tout en maintenant la majeure partie du chlorure de vinyle, du 1-chloro-1-fluoroéthane et du fluorure d'hydrogène sous forme liquide. On travaille généralement à des températures comprises par exemple entre 60 et 120 °C. Des températures comprises entre 80 et 110 °C se sont révélées avantageuses. On travaille généralement à des pressions comprises par exemple entre 2 et 30 bar. Des pressions comprises entre 5 et 20 bar se sont révélées avantageuses.

La durée de la réaction nécessaire pour assurer un rendement optimal en 1-chloro-1-fluoroéthane et/ou en 1,1-difluoroéthane est variable en fonction des conditions opératoires et, en pratique, peut être évaluée facilement par voie expérimentale. Lorsque le procédé est effectué en continu, le temps de séjour conventionnel du chlorure de vinyle dans le réacteur, c'est-à-dire le rapport entre le volume de mélange réactionnel contenu dans le réacteur et le débit total de chlorure de vinyle et de fluorure d'hydrogène à l'état liquide est généralement de 0,1 à 5 heures.

Le procédé selon l'invention peut être réalisé dans tout réacteur réalisé dans un matériau résistant à la température et à la pression de travail et résistant au fluorure d'hydrogène dans les conditions de travail du procédé. On utilise avantageusement des réacteurs réalisés en acier au carbone, en acier inoxydable ou en alliages tels que ceux connus sous les marques **MONEL**, **INCONEL** ou **HASTELLOY**. On peut également utiliser des réacteurs munis d'un revêtement en un métal ou alliage résistant au fluorure d'hydrogène, ou recouverts d'une couche d'une résine inerte dans les conditions de réaction, notamment des résines fluorées.

Dans le procédé selon l'invention, le fluorure d'hydrogène et le chlorure de vinyle peuvent être introduits dans le mélange réactionnel indifféremment à l'état liquide ou à l'état vapeur. En raison de la présence du solvant organique, le chlorure de vinyle peut être introduit dans le réacteur même à l'état liquide sans entraîner la formation de quantités importantes de sous-produits lourds. Le procédé selon l'invention présente l'avantage appréciable d'un productivité largement supérieure à celle permise par le procédé antérieur connu dans lequel le chlorure de vinyle est introduit sous forme gazeuse dans un milieu liquide constitué essentiellement de fluorure d'hydrogène, tout en limitant la formation de sous-produits lourds. Il permet d'obtenir d'excellentes sélectivités et rendements en 1-chloro-1-fluoroéthane et 1,1-difluoroéthane, bien supérieurs à ceux obtenus dans les procédés antérieurs connus. En outre, selon les conditions réactionnelles mises en oeuvre et la nature du catalyseur éventuellement utilisé, il permet de moduler dans une large mesure le rapport quantitatif entre le 1-chloro-1-fluoroéthane et le 1,1-difluoroéthane formés.

Les exemples qui suivent sont donnés dans le but d'illustrer l'invention mais ne sont nullement limitatifs.

### Exemple 1 (Comparaison)

Après avoir été mis sous vide et refroidi à -20 °C, un autoclave en acier inoxydable, d'une capacité de 0,5 l, équipé d'un agitateur, d'une sonde de température, d'un tube plongeant permettant d'effectuer des prélèvements en phase liquide et d'une entrée pour l'introduction des réactifs, a été successivement chargé avec 127 g de chlorure de vinyle (VC) liquide, puis avec 84 g de fluorure d'hydrogène (HF) liquide, de manière à obtenir un rapport molaire HF / VC égal à 2. L'autoclave a ensuite été plongé dans un bain d'huile préchauffé à température adéquate, de manière à amener le mélange réactionnel liquide, maintenu sous agitation, à la température de 60 °C. Le mélange réactionnel a été maintenu à cette température sous pression autogène pendant une heure. Les principales conditions opératoires et les résultats d'analyse par chromatographie en phase gazeuse (CPV) d'un échantillon prélevé hors du mélange réactionnel liquide sont rassemblés dans le tableau I. La colonne "Conv. VC" exprime le degré de conversion du VC, c'est-à-dire la quantité de VC transformée par rapport à la quantité de VC mise en oeuvre. Pour les différents produits formés (151a = 1-chloro-1-fluoroéthane; 152a = 1,1-difluoroéthane; 11DCE = 1,1-dichloroéthane; lourds = sous-produits lourds), la sélectivité correspond à la quantité de VC transformée en ce produit par rapport à la quantité totale de VC transformée. Cet exemple montre que, dans les conditions du brevet DE 859887, c'est-à-dire avec une teneur initiale en VC dans le mélange réactionnel égale à 100 % molaire des composés organiques, des sous-produits lourds sont formés en quantité très importante.

### Exemple 2 (Comparaison)

Un essai a été réalisé à 30 °C, dans le même appareil que celui mis en oeuvre dans l'exemple 1, au départ d'un mélange réactionnel liquide constitué uniquement de VC et de HF, avec un rapport molaire HF / VC de 50. Les résultats obtenus après une heure à 30 °C sont présentés au tableau I. Cet exemple montre que, en absence d'hydrocarbure halogéné saturé dans le mélange réactionnel liquide initial, la mise en oeuvre d'un excès très important de fluorure d'hydrogène n'empêche pas la transformation d'une fraction importante du VC en sous-produits lourds.

### Exemple 3 (conforme à l'invention)

Un essai a été réalisé à 60 °C, dans le même appareil que celui mis en oeuvre dans les exemples précédents, au départ d'un mélange réactionnel liquide constitué de VC, de 1,1-dichloroéthane et de HF, avec un rapport molaire HF / VC / 11DCE de 10/1/10. Les résultats obtenus après 2 heures à 60 °C sont présentés au tableau I. Par comparaison avec les exemples 1 et 2, l'exemple 3 selon l'invention montre que, lorsque les réactifs sont dilués dans un hydrocarbure halogéné dès le démarrage du procédé, la quantité de sous-produits lourds formés est fortement réduite. En outre, cet exemple montre, qu'en absence de catalyseur, une sélectivité très élevée en 1-chloro-1-fluoroéthane est obtenue.

### Exemple 4 (Comparaison)

Un essai a été réalisé à 60 °C, dans le même appareil que celui mis en oeuvre dans les exemples précédents, au départ d'un mélange réactionnel liquide constitué de VC et de HF, avec un rapport molaire HF / VC de 3/1, en présence de 0,05 mole de SnCl₄ par mole de VC. Les résultats obtenus après 2 heures de réaction à 60 °C sont présentés au tableau I. Cet exemple montre que, dans des conditions similaires à celles du brevet US 2495407, c'est-à-dire avec une teneur initiale en VC dans le mélange réactionnel liquide égale à 100 % molaire des composés organiques et en présence de SnCl₄ comme catalyseur, des sous-produits lourds sont formés en quantité très importante.

### Exemple 5 (conforme à l'invention)

L'essai de l'exemple 4 a été répété en présence de 1,2-dichloroéthane (12DCE) comme hydrocarbure halogéné (rapport molaire HF / VC / 12DCE = 12/1/9). Au début, la réaction conduit principalement à la formation de HFA-151a (sélectivité de près de 80 % dans les premières minutes). La teneur en HFA-151a diminue ensuite au profit de la formation du HFA-152a. La formation de lourds reste limitée. Après 4 heures de réaction, la conversion du VC est complète. Les résultats obtenus après 4 heures de réaction à 60 °C sont présentés au tableau I.

### Exemples 6 à 12 (conformes à l'invention)

Une série d'essais a été réalisée à 80 °C, dans le même appareil que celui mis en oeuvre dans les exemples précédents, au départ d'un mélange réactionnel liquide constitué de VC, de HF, de 1,2-dichloroéthane comme hydrocarbure halogéné et de différents catalyseurs d'hydrofluoration. Les conditions opératoires détaillées et les résultats sont rassemblés dans le tableau I. Ces exemples illustrent l'influence de la nature du catalyseur sur la distribution quantitative des produits obtenus. En absence de catalyseur ou en présence de tétrafluorure de vanadium, de trifluorure de chrome ou de trichlorure de vanadium, le principal produit formé est le 1-chloro-1-fluoroéthane. En présence de tétrachlorure d'étain, de pentachlorure de molybdène ou d'hexachlorure de tungstène, des quantités plus importantes de 1,1-difluoroéthane sont formées. Dans tous les cas, la quantité de sous-produits lourds reste faible. Selon le catalyseur mis en oeuvre, le procédé permet donc de moduler dans une large mesure le rapport entre le 1-chloro-1-fluoroéthane et le 1,1-difluoroéthane formés.

### Exemples 13 et 14 (conformes à l'invention)

L'essai de l'exemple 5 a été répété en présence de quantités différentes de tétrachlorure d'étain. Les résultats obtenus après 4 heures de réaction à 60 °C sont présentés au tableau I. Dans l'essai de l'exemple 14, après 6 heures à 60 °C, la température du réacteur a ensuite été portée à 80 °C. Après 1 heure à cette température, la distribution des produits était la suivante : HFA-151a : 10 %; HFA-152a : 65 %; 11DCE : 16 %; sous-produits lourds : 9 %. La comparaison de ces exemples avec les exemples 5 et 11 enseigne qu'il est également possible de moduler la répartition entre le 1-chloro-1-fluoroéthane et le 1,1-difluoroéthane formés en ajustant la quantité de catalyseur dans le solvant et/ou la température. La quantité de 1,1-difluoroéthane formée augmente au détriment de la quantité de 1-chloro-1-fluoroéthane formée lorsque la quantité de catalyseur augmente ou lorsque la température augmente, sans provoquer de variation importante de la quantité de sous-produits lourds formés.

### Exemples 15-19 (conformes à l'invention)

Les essais suivants ont été réalisés en continu, à une température de 90 °C et sous une pression de 15 bar, dans un réacteur en acier inoxydable de 200 cm³, muni d'un agitateur, d'une double enveloppe dans laquelle circule une huile permettant de chauffer le réacteur et d'un tube de soutirage par débordement, maintenu à la même température que le réacteur. Le chlorure de vinyle, le fluorure d'hydrogène et le catalyseur ont été introduits en continu dans le réacteur, contenant initialement du 1-chloro-1-fluoroéthane comme hydrocarbure halogéné, maintenu sous forme liquide à la température de réaction par régulation de la pression dans le réacteur. Après 5 heures de mise en régime, des prélèvements de la phase gazeuse et de la phase liquide débordant de l'autoclave ont été effectués. Après détente à pression atmosphérique et neutralisation dans un scrubber de lessive caustique 0,1 molaire, l'effluent a été analysé par chromatographie en phase gaz. Les conditions opératoires et les résultats obtenus sont repris dans le tableau II. Les exemples 15 à 19 démontrent l'intérêt tout particulier de réaliser le procédé selon l'invention en continu, en particulier pour limiter drastiquement la formation de sous-produits lourds. En outre, la comparaison des résultats des exemples 15 et 16 montre que la quantité de 1,1-difluoroéthane formée augmente au détriment du 1-chloro-1-fluoroéthane lorsque le temps de séjour augmente. La comparaison des résultats des exemples 15 et 17 montre le même effet lorsque la concentration en catalyseur augmente. La comparaison des résultats des exemples 15 et 18 d'une part et des exemples 16 et 19 d'autre part montre le même effet lorsque le rapport HF/VC augmente. En aucun cas, une modification de ces paramètres ne provoque une variation importante de la quantité de sous-produits lourds formés.

**TABLEAU I**

| Ex. | Composition du mél. réactionnel (HF / VC / HC*) (rapport mol.) | Catalyseur (mole/ /mole VC) | Temp. de consigne (°C) | Durée à temp stable (h) | Conv. VC (%) | Sélectivités (% VC converti) | | | |
|---|---|---|---|---|---|---|---|---|---|
| | | | | | | 151a | 152a | 11DCE | Lourds |
| 1(C) | 2 / 1 / 0 | - | 60 | 1 | 95 | 59 | < 1 | 5 | 35 |
| 2(C) | 50 / 1 / 0 | - | 30 | 1 | 97 | 50 | < 1 | 4 | 46 |
| 3 | 10 / 1 / 10 | - | 60 | 2 | 89 | 91 | < 1 | nm* | 8 |
| 4(C) | 3 / 1 / 0 | 0,05 SnCl₄ | 60 | 2 | 100 | 12 | 4 | 59 | 25 |
| 5 | 12 / 1 / 9 | 0,05 SnCl₄ | 60 | 4 | 100 | 40 | 15 | 41 | 4 |
| 6 | 11 / 1 / 8 | - | 80 | 2 | 99 | 84 | 2 | 2 | 12 |
| 7 | 9 / 1 / 9 | 0,05 VF₄ | 80 | 1 | 99 | 89 | 1 | 2 | 8 |
| 8 | 11 / 1 / 9 | 0,05 CrF₃ | 80 | 2 | 99 | 89 | 1 | 2 | 8 |
| 9 | 11 / 1 / 9 | 0,05 VCl₃ | 80 | 2 | 99 | 88 | 1 | 3 | 8 |
| 10 | 12 / 1 / 10 | 0,05 WCl₆ | 80 | 4 | 100 | 48 | 11 | 28 | 13 |
| 11 | 12 / 1 / 10 | 0,05 SnCl₄ | 80 | 4 | 100 | 23 | 33 | 33 | 11 |
| 12 | 12 / 1 / 10 | 0,05 MoCl₅ | 80 | 4 | 100 | 28 | 27 | 38 | 7 |
| 13 | 12 / 1 / 9 | 0,01 SnCl₄ | 60 | 4 | 100 | 70 | 6 | 12 | 12 |
| 14 | 11 / 1 / 9 | 0,24 SnCl₄ | 60 | 4 | 100 | 21 | 34 | 40 | 5 |

| | | | | | | | | | |
|---|---|---|---|---|---|---|---|---|---|
| * : HC = hydrocarbure halogéné saturé | | | | | | | | | |
| * : nm = non mesuré | | | | | | | | | |

**TABLEAU II**

| N° Ex. | Temps séjour (h) | Rapport HF/VC/SnCl4 (mol/mol/mol) | Conversion VC (%) | Sélectivités (% VC converti) | | | | Rapport 152a/151a |
|---|---|---|---|---|---|---|---|---|
| | | | | 151a | 152a | 11DCE | Lourd | |
| 15 | 1 | 2,8/1/0,007 | 99,3 | 29 | 52 | 18 | 0,9 | 1,79 |
| 16 | 0,38 | 2,5/1/0,007 | 92 | 43 | 42 | 14 | 0,4 | 0,98 |
| 17 | 1 | 3/1/0,003 | 99,6 | 42 | 31 | 26 | 1,2 | 0,74 |
| 18 | 1 | 1,6/1/0,007 | 99,4 | 47 | 30 | 21 | 1,8 | 0,64 |
| 19 | 0,38 | 6,2/1/0,007 | 99,7 | 24 | 65 | 10 | 0,9 | 2,71 |

## Revendications

1. Procédé de fabrication de 1-chloro-1-fluoroéthane et/ou de 1,1-difluoroéthane par réaction entre du fluorure d'hydrogène et du chlorure de vinyle en phase liquide, caractérisé en ce qu'on introduit le fluorure d'hydrogène et le chlorure de vinyle dans un solvant organique constitué d'au moins un hydrocarbure halogéné saturé.

2. Procédé selon la revendication 1, caractérisé en ce que l'hydrocarbure halogéné saturé est sélectionné parmi les hydrocarbures chlorés, fluorés ou chlorofluorés contenant de 1 à 8 atomes de carbone.

3. Procédé selon la revendication 2, caractérisé en ce qu'on utilise comme solvant un hydrocarbure halogéné saturé du procédé ou un mélange d'hydrocarbures halogénés produit dans le procédé.

4. Procédé selon une quelconque des revendications 1 à 3, caractérisé en ce que le mélange réactionnel contient à tout moment au moins 55 % en poids de solvant.

5. Procédé selon l'une quelconque des revendications 1 à 4, caractérisé en ce qu'on règle l'introduction du chlorure de vinyle et du fluorure d'hydrogène pour que, à tout moment, la teneur en chlorure de vinyle soit inférieure à 15 % et celle en fluorure d'hydrogène inférieure à 30 % du poids du mélange réactionnel.

6. Procédé selon une quelconque des revendications 1 à 5, caractérisé en ce que le rapport molaire entre le fluorure d'hydrogène et le chlorure de vinyle mis en oeuvre est d'au moins 1 et ne dépasse pas 20.

7. Procédé selon l'une quelconque des revendications 1 à 6, caractérisé en ce qu'on effectue la réaction en présence d'un catalyseur d'hydrofluoration choisi parmi les dérivés des métaux des groupes IIIa, IVa, IVb, Va, Vb et VIb du tableau périodique des éléments et leurs mélanges, de préférence parmi les dérivés de l'étain.

8. Procédé selon une quelconque des revendications 1 à 7, caractérisé en ce que la réaction est réalisée à une température au moins de 40 °C et ne dépassant pas 130 °C et sous une pression au moins égale à 2 bar et ne dépassant pas 50 bar.

9. Procédé selon une quelconque des revendications 1 à 8, caractérisé en ce qu'on soutire en continu le produit recherché hors du mélange réactionnel.

10. Procédé selon la revendication 9, appliqué à la production du 1,1-difluoroéthane, caractérisé en ce qu'il est soutiré sous forme gazeuse.

## Claims

1. Process for the manufacture of 1-chloro-1-fluoroethane and/or 1,1-difluoroethane by reaction between hydrogen fluoride and vinyl chloride in the liquid phase, characterized in that the hydrogen fluoride and the vinyl chloride are introduced into an organic solvent consisting of at least one saturated halogen-containing hydrocarbon.

2. Process according to Claim 1, characterized in that the saturated halogen-containing hydrocarbon is selected from chloro-, fluoro- or chlorofluorohydrocarbons containing from 1 to 8 carbon atoms.

3. Process according to Claim 2, characterized in that a saturated halogen-containing hydrocarbon of the process or a mixture of halogen-containing hydrocarbons of the process is used as solvent.

4. Process according to any one of Claims 1 to 3, characterized in that the reaction mixture contains, at all times, at least 55% by weight of solvent.

5. Process according to any one of Claims 1 to 4, characterized in that the introduction of the vinyl chloride and hydrogen fluoride is controlled so that, at all times, the vinyl chloride content is less than 15% and that of hydrogen fluoride is less than 30% of the weight of the reaction mixture.

6. Process according to any one of Claims 1 to 5, characterized in that the molar ratio between the hydrogen fluoride and the vinyl chloride used is at least 1 and does not exceed 20.

7. Process according to any one of Claims 1 to 6, characterized in that the reaction is carried out in the presence of a hydrofluorination catalyst chosen from derivatives of metals of groups IIIa, IVa, IVb, Va, Vb and VIb of the Periodic Table of the elements, and their mixtures, preferably chosen from tin derivatives.

8. Process according to any one of Claims 1 to 7, characterized in that the reaction is performed at a temperature of at least 40°C and not exceeding 130°C and at a pressure at least equal to 2 bar and not exceeding 50 bar.

9. Process according to any one of Claims 1 to 8, characterized in that the desired product is withdrawn continuously from the reaction mixture.

10. Process according to Claim 9, applied to the production of 1,1-difluoroethane, characterized in that it is withdrawn in gaseous form.

## Patentansprüche

1. Verfahren zur Herstellung von 1-Chlor-1-fluorethan und/oder 1,1-Difluorethan durch Reaktion zwischen Fluorwasserstoff und Vinylchlorid in flüssiger Phase, dadurch gekennzeichnet, daß man den Fluorwasserstoff und das Vinylchlorid in ein organisches Lösungsmittel gibt, das aus wenigstens einem gesättigten Halogenkohlenwasserstoff besteht.

2. Verfahren gemäß Anspruch 1, dadurch gekennzeichnet, daß der gesättigte Halogenkohlenwasserstoff unter den Chlor-, Fluor- oder Chlorfluorkohlenwasserstoffen mit 1 bis 8 Kohlenstoffatomen ausgewählt ist.

3. Verfahren gemäß Anspruch 2, dadurch gekennzeichnet, daß man als Lösungsmittel einen gesättigten Halogenkohlenwasserstoff des Verfahrens oder ein Gemisch von Halogenkohlenwasserstoffen, das bei dem Verfahren hergestellt wird, verwendet.

4. Verfahren gemäß einem der Ansprüche 1 bis 3, dadurch gekennzeichnet, daß das Reaktionsgemisch stets wenigstens 55 Gew.-% Lösungsmittel enthält.

5. Verfahren gemäß einem der Ansprüche 1 bis 4, dadurch gekennzeichnet, daß man die Zugabe des Vinylchlorids und des Fluorwasserstoffs einstellt, damit stets der Gehalt an Vinylchlorid kleiner ist als 15% und der an Fluorwasserstoff kleiner als 30% des Gewichts des Reaktionsgemischs.

6. Verfahren gemäß einem der Ansprüche 1 bis 5, dadurch gekennzeichnet, daß das eingesetzte Molverhältnis zwischen Fluorwasserstoff und Vinylchlorid wenigstens 1 ist und 20 nicht übersteigt.

7. Verfahren gemäß einem der Ansprüche 1 bis 6, dadurch gekennzeichnet, daß man die Reaktion in Gegenwart eines Hydrofluorierungskatalysators ausführt, der unter den Derivaten der Metalle der Gruppen IIIa, IVa, IVb, Va, Vb und VIb des Periodensystems der Elemente und deren Gemischen, vorzugsweise unter den Derivaten des Zinns ausgewählt ist.

8. Verfahren gemäß einem der Ansprüche 1 bis 7, dadurch gekennzeichnet, daß die Reaktion bei einer Temperatur, die wenigstens 40 °C ist und 130 °C nicht übersteigt, und unter einem Druck, der wenigstens gleich 2 bar ist und 50 bar nicht übersteigt, durchgeführt wird.

9. Verfahren gemäß einem der Ansprüche 1 bis 8, dadurch gekennzeichnet, daß man das gesuchte Produkt kontinuierlich aus dem Reaktionsgemisch entnimmt.

10. Verfahren gemäß Anspruch 9, angewendet auf die Herstellung von 1,1-Difluorethan, dadurch gekennzeichnet, daß es in Gasform entnommen wird.
